# EUROPEAN PATENT APPLICATION

(11) **EP 1 500 741 A1**
(43) Date of publication of application: **26.01.2005**
(21) Application number: 04103482.8
(22) Date of filing: 21.07.2004
(51) Int. Cl.: D06M 16/00, D06M 13/00, G01N 33/36, G01N 33/543

(54) **Labelled fibres and immunochemical methods for their revelation**

(30) Priority: 21.07.2003 IT MI20031483
(71) Applicant: Bartholdy-Consultadoria e Servicos Lda, 9000 Funchal, Madeira (PT)
(72) Inventor: Bizzini, Bernard, 81000 ALBY (FR); Volpato, Ivo, 06070 SAN MARIANO (IT); Lippmann, Marco, 6957 ROVEREDO / TI (CH)
(74) Representative: Gervasi, Gemma, Dr.

(57) **Abstract**

The present invention relates to a method for covalently coupling haptenic radicals to fibres or natural or synthetic yarns to allow fibres, yarns, fabrics and non-wovens to be traced back. Coupling of the tracer substance to these materials can be carried out either directly or through a linking bridge.

A further objective of the invention is constituted by labelled fibres or yarns as originating from such a procedure.

A still further scope of the invention is a method for revealing tracer substances coupled to fibres and, as a consequence, to yarns, fabrics and non-wovens as performed by means of immunochemical methods.

## Description

### FIELD OF THE INVENTION

The field of the invention relates to immunochemical methods to trace back the origin of fibres, yarns, fabrics and non-wovens.

### FORMER ART

Various types of fibres and materials are used in textile industry for manufacturing yarns, fabrics and finished materials. Two types of fabrics are known: the so-called fabrics and the non-woven, the latters consist of mixtures of polymers with fibres of varying origins and are utilized for the most varying purposes (seat and sofa spreads, stocking-stoles, carpets, sanitary facing, clothing). They are characterized by the fact that they are not subjected to spinning, but manufactured by the use of other known industrial processes, such as "drylaid", "spunlaid", "wetlaid" or "mechanical bonding".

Whether dealing with true fabrics or with non-wovens, a technical problem exists: which concerns the need to identify the origin, composition and authenticity of the fibres composing fabrics and non-wovens.

Such a scope can be reached by labelling fibres.

It is well known that various additives can be mixed with polymers which are subsequently subjected to various processes of manufacturing. A few additives, however, exhibit shortcomings: in the case of some dyeings, in fact, residues are left in the processing machines and in the successive cycles of manufacturing these residues will settle on the fibres, even mix themselves with the new and different dye. Consequently, it is necessary to have labels which once incorporated in the fibres are not visible to the eye.

Concerning fibres or manufactured items labelled with tracers meeting this requirement, in the state of art is reported only coupling with fluorescent tracers. In particular, the patent WO 9963145 gives references to fluorescent tracers (cyanines) coupled to fibres by means of cross-linking agents as well as to a revelation method using radiations of defined wave-lengths to which the tracers are visible. However, such a method is not very selective and presents main drawbacks consisting in the fact that bleaching and/or fluorescent agents in textile industries are very often used to confer specific characteristics on the manufactured items. Of course, in these cases fluorescent tracers cannot be used. Not strictly linked to the textile industry, but routinely used in clinical-biochemical practice are the ELISA systems (Enzyme Linked Immunosorbent Assay).

These systems are used for researching entities of varying nature, whether haptenic or macromolecular and their sensitivity is very high, since they allow to determine concentrations of the analytes at level of nanomicro-grams; furthermore, these systems are highly specific.

### SUMMARY

The present invention relates to a method for covalently coupling haptenic radicals to fibres or natural or synthetic yarns so as to allow to trace back the fibre even when mixed with various amounts of unlabelled analogous fibre and even when subjected to spinning, weaving or various other processes for manufacturing fabrics and non-wovens.

Coupling of the label to fibre or yarns can take place directly or through a bonding arm exhibiting a polyaminoacid or polyamine structure or an aminopolyacid or polyacid structure.

Revelation of the labels linked to the fibre and, as a consequence, to yarns and fabrics or to non-wovens is performed by immunochemical methods, preferably ELISA, with anti-label antibodies (polyclonal or monoclonal) which are successively revealed preferably with specific anti-antibodies to which an enzyme is coupled or, alternatively, by using anti-label antibodies (polyclonal or monoclonal) directly coupled to an enzyme. Such an enzyme by reacting with a convenient substrate, develops a specific colour in yarns, fabric, non-wovens, revealing the presence and/or the quantity of labelled fibre.

Given that in these systems there is also a coloration of the solution in which the tested yarns / fabrics / non-wovens are suspended, the presence of the label is detectable even after dyeing of fabrics / non-wovens.

This allows a visual, rapid and simple characterization of the origin and nature of the product without using any apparatus as well as the quantitative determination of the colour intensity (proportional to the quantity of label present) testifying the genuine character of the product when a colorimeter is used.

### DETAILED DESCRIPTION OF THE INVENTION

Object of the present invention is a method for labelling fibres or natural and synthetic yarns to which a label is covalently coupled and the relevant identification procedure.

The present invention concerns also natural fibres and/or synthetic polymers and / or natural / synthetic mix yarns to which such tracers consisting of substances with haptenic structure detectable means of immunochemical methods are coupled.

The detection method of natural and / or synthetic fibres, yarns and / or fabrics to which such tracers, preferably represented by substances exhibiting a haptenic structure, are coupled, represents a further aspect of the present invention. Together they represent a specific and simple method of identification of the origin and authenticity of the fibres, yarns, fabrics, non-wovens and of fibre items in general.

The labelling method comprises two steps: activation of the fibre and coupling of the label to the pre-activated fibre (labelling).

Various types of fibres, natural or synthetic are used. According to the present invention among natural fibres are preferred cotton, linen, hemp, wool, silk and among synthetic ones, nylon and rayon. Still more preferred are cotton and nylon.

A pre-activation step may be performed depending on both the fibre and label type. In one of its preferred aspects when the fibre is natural and is preferably cotton, the pre-activation consists of a pre-oxidation of fibres with sodium periodate; fibre is previously wetted with water, preferably in a reactor under pressure (3 bars). After removing the excess of water the pre-activated fibre is treated with 0.05 M sodium periodate solution for activation, preferably for at least 5 hours, at a temperature in the range of 35 - 50°C, preferably of 40 - 45 °C; for about 400 g fibres, preferably cotton, are used 10 Lt of solution. Eventually it is washed with water.

In case of a the labelling of natural or synthetic fibres with tracers of carbohydrate nature, the tracer substance is also pre-activated with sodium periodate.

In case of a later coupling with tracer substances or with chemical bridges containing polyamines or sulfhydryl compounds, the fibre activation comprises a step of wetting with water and in the treatment with about 10 Lt of a solution containing DVS (divinylsulfone) at a 0.01 - 0.05 % concentration in 0.05 M carbonate / bicarbonate buffer, pH 11, followed by washing with water.

Nylon fibres are, in contrast, pre-activated by a treatment in acidic conditions at a pH lower than 5, preferably with 3.5 N HCl for at least 16 hours, followed by at least one washing with water.

Pre-activated fibres can then be labelled with the tracer substance. Labelling can also be carried out on yarns.

Labelling is performed by covalent coupling of the tracer substance, preferably with a haptenic structure, to fibres. The substances used as tracer exhibit various chemical structures and meet defined requirements.

The tracer substance is stable over time and over various treatments to which the fiber may be subjected during the manufacturing process (spinning, degumming, bleaching, dyeing, weaving, smoothing); it shows a high reactivity with the fibre, i.e. coupling does not result in any alteration or masking of the characteristic molecular determinant necessary for the later immunological revelation; it is easy to be detected by a specific antibody (polyclonal or monoclonal) and is cheap; moreover, commercial properties of yarns and fabrics are not impaired and the tracer substance does neither interfere with usual treatments to which fibre is subjected nor does it bring about toxic or biopharmacological effects at the level of skin and mucous membranes to which it comes in contact.

The tracer substance according to the present invention is chosen among the group constituted by aminoacids, carbohydrates, synthetic inert substances bearing reactive groups of amine, amide, aldehyde, carboxylic or hydroxylic types.

Tracer substances used as labels meeting the above - mentioned requirements are preferably selected from the group of carbohydrates, between maltose and polygalacturonic acid; from the group of aminoacids: between GABA (gamma-amino-butyric acid) and DL-aspartic acid; from the group of carboxylic acids: among nicotinic acid, m-toluic acid, urocanic acid, salicylic acid, cholic acid; from amino-compounds: among: 1-naphtylamine, 5-amino-uracile, 6-amino-purine, triaminophenol; from the group of purine bases; 6-methyl-purine; from the group of sulfhydryl compounds: 2-mercaptobenzimidazole; from the group of aldehydes: 9-anthraldehyde; from the group of amides nicotinamide which is preferred.

Coupling of the tracer substance to natural and synthetic fibres and, among the latters preferably nylon and rayon, is carried out taking advantage of aldehyde groups generated on the fibre and of amine groups present on the tracer substance, followed by reduction in order to convert imino into amino bonds. Alternatively other methods can be used such as those utilizing DVS (divinylsulfone) or EDAC (1-ethyl-3(3-dimethylaminopropyl)carbodiimide). DVS is preferably used for coupling triaminophenol and 2-mercaptobenzimidazole; carbodiimide for coupling aminoacids, cholic acid or compounds bearing at least one carboxylic group.

Activated synthetic fibres, in contrast, are labelled by allowing them to react with the various tracer substances using reaction schedules depending on the specific reactive tracer radical.

In this case DVS is preferably used for coupling nicotinamide, 2-mercaptoimidazole and uronic acid, whereas carbodiimide is used for coupling aminoacids and nicotinic acid.

Generally fibres (about 400 gr natural fibres or 500 gr synthetic fibres) are suspended in about 10 Lt of solution with pH < 7 or pH > 7 depending on the nature of the coupling reaction to be carried out. The solution preferably contains also the tracer substance to be coupled.

Solutions at pH < 7, preferably at pHs in the range of 5 to 7 including 0.1 M acetate or phosphate buffer are prefer used for coupling with EDAC. Solutions at pH > 7, preferably at a pHs comprised from 8 to 10 contain, instead, 0.05 M carbonate / bicarbonate buffer or Na2HP04 solutions and are used for coupling other types of tracer substances to the two types of fibres, natural or synthetic, to activate the reaction with DVS or that between aldehyde and amino groups.

Such solutions contain, in addition to buffers also substances which act by activating fibres or tracer substances. These fibre activators are prefer glutaraldehyde and DVS. Among tracer activators is preferably used NalO4.

After suspending fibres for at least 2 hours in the above-mentioned solutions at room temperature, they are washed with about 10 Lt of water and dryied in the oven at about 60 °C (in the case of natural fibres) or at about 40 °C (in the case of synthetic fibres).

The bond of tracer substance to the fibre can be direct or take place through bridges of polyaminoacids, polyamines, aminopolyacids or polyacids. The use of such bridges allows to increase the sensitivity of a further detection reaction with the antibody. The use of multiple chemical bridges allows also the amplification of the number of tracer molecules coupled to the fibre and the possibility to couple different tracer molecules to one single fibre. Among bridges of polyamines is preferably used triaminophenol, whereas among those of polyaminoacids, polylysine, polyglutamic and polyaspartic acids. Cotton derivatized with polylysine can be used for binding at least another tracer substance either through one of its amino groups, or through one of its carboxylic groups. In the case of polyamine arms bound to cotton, tracers are coupled to bridges by means of a reaction with carbodiimide, when carboxylic groups are present, or by a reaction involving aldehyde groups when the tracer substance can be oxidized. The presence in the tracer of halogenic radicals allows its direct binding to amino groups.

To the aim of producing labelled yarns, fabrics and non-wovens it is possible to use only labelled fibres and the label may be equal or different. Preferably fibres labelled with various tracer substances, are mixed homogeneously with virgin, non labelled fibres. The percentage of labelled fibre ranges from 0.01 to 10 %, preferably from 0.1 to 5 %, and more preferably 1 to 3 % and still more preferably at least 1 % (for fabrics) and between 1 and 10 %, preferably 2 to 7 %, more preferably 2.5 to 6 % and still more preferably at least 3 % (for non-woven), over total fibres. The concentration of labelled fibre needed to obtain a detectable signal varies, as a matter of fact, according to the tracer nature, the relative response of the specific antibody and the sensitivity of the revelation method. Homogeneization of labelled and unlabelled fibres is carried out prior to threading, in the case of fabrics, and prior to successive manufacturing steps in the case of non-wovens, so as to achieve a uniform distribution of the labelled fibre either in the yarns, or fabrics, or in the manufactured item, which, as a consequence, is also labelled.

In the case of items manufactured with mixed fibres (for instance cotton / nylon) it is sufficient to homogeneize only one type of labelled fibre.

As said above, in the preparation of yarns it is possible to homogeneize labelled fibre with different tracer substances: in this case a multiple labelling of finished fabrics is obtained that can readily be revealed. The revelation method of labelling relies on immunochemical methods. Several ELISA methodologies may be used in relation to the scope for which the method is calibrated: generally the methods provide for the binding of an antigen (the analyte to be tested) to a support (solid phase), the successive addition of a specific antibody and the revelation by an anti-antibody to which an enzyme is coupled (A. Voller and D. Bidwell, The Enzyme Linked Immunosorbent Assay, 1980, vol. II, Microsystem Ltd Publ., Guernsey, G.B.). The enzyme usually performs a chromogenic reaction which is measured.

The solid phase can be of different nature, for instance nitrocellulose or nylon. For the detection of substances with haptenic structure coupled to fibres, it is necessary to have the corresponding specific antibodies available. In some cases the antibodies are commercial; such as, for instance, anti-GABA antibodies or the anti-aspartate antibodies. In other cases, antibodies can be obtained by immunizing animals with immunogens corresponding to the various tracer substances, according to the know methods. The immunogens can be prepared using the tracer substances of interest (as reported in experimental examples father on).

After producing and purifying the antibodies according to normally used methodologies (R. Scopes, Protein purification, Principles and Practice, 1982, Springer-Verlag Publ., NY) they are coupled with a revealing enzyme when a direct ELISA system is used. When the indirect method is used the enzyme is coupled to an anti-antibody, namely to a second antibody, directed to the anti-label antibody.

The general procedure for revealing the tracer substance foresees, in agreement with known methodologies (A. Johnstone and R. Thorpe, Immunochemistry in Practice, 1982, Blackwell Sci. Publ., Oxford), that a fragment (about 1 - 2 gr) of yarns, or fabrics, or non-wovens in which the label is present, is immersed, after saturation of the still possible free sites with an heterologous protein, into a solution containing the antibody (polyclonal or monoclonal) specific for the label and it is left in contact for 2 hours. Following the fixation of the antibody directed to the label, the fragment is withdrawn, exhaustively washed and then immersed into a solution containing the anti-specific antibody labelled with enzyme, directed to the first one.

Finally, the fragment of fabrics or of non-wovens is repeatedly washed. The quantity of the second antibody labelled with an enzyme and directed to the first antibody which itself has reacted with the tracer substance is revealed by having the enzyme react with the appropriate substrate. For this purpose, the fragment is immersed into a solution containing the substrate for the enzyme.

Alternatively, according to known methods, if the antibody specific for the tracer substance is directly labelled with an enzyme, the revelation method includes but one immersion of the fabrics fragment into the solution of labelled antibody, a subsequent washing and, then, the direct immersion into a solution containing the enzyme substrate. In both cases, however, the solution will develop a coloration proportional to the quantity of labelled antibody and consequently to that of the tracer substance present on the fibre or fabric.

Colour development deriving from reaction of the revealing enzyme with its substrate is, in effect, the sign of the label presence thus allowing the origin of yarns to be identified already by a simple visual examination without using an instrument.

Besides this qualitative determination, this methodology also permits a quantitative determination of the tracer present by surface unit of fabric; this is possible by determining spectrophotometrically the intensity of the coloration of the liquid containing the substrate for the enzyme by comparison with that of a standard reference solution, according to the practice of the immunoenzymatic methods known by the art.

Determination of the amount of label present in one surface unit of yarns or fabric permits the evaluation of the authenticity of the product.

The choice of the revealing enzymes is not by itself compelling: in fact, are used various enzymes, for instance, alkaline phosphatase, peroxidase (HRP) and beta-galactosidase.

Each of these can react with a series of varying substrates which also develop varying colorations. A few examples of substrates, all of them well known and purchasable are: for HRP O-phenylene-diamine (with development of a coloration brown/orange), O-dianisidine (with development of a coloration yellow/orange), 5-aminosalicylic acid (with development of a coloration brown), 2,2'-azino-bis-(3-ethylbenzothiazoline-6-sulfonate) (with development of a coloration green), tetramethylbenzidine (with development of a coloration blue); for beta-galactosidase: nitrophenyl-beta-D-galactoside (with development of a yellow coloration); for alkaline phosphatase: p-nitrophenylphosphate (with development a yellow coloration). The existence of this palette of revelation colours is useful in cases in which fabric or non-woven has undergone a dyeing process whose colour could interfere with the optical revelation of the tracer. Therefore, it is possible to vary the combination enzyme-substrate according to the coloration of the fabric / non-woven to be tested.

As to the quantitative determination, instead, there are no particular problems in that the spectrophotometric measurement is carried out directly on the liquid containing the substrate. Revelation methodology is common for all tracer substances coupled to varied fibers (natural, synthetic, mixed); however, so as to have good responses, a preliminary calibration is necessary, and is obtained by varying the percentage of labelled or non labelled fiber according to the standard.

In agreement with the present invention, it is possible to reveal the nature, origin and authenticity preferably of the following products:
a. yarns and fabrics of cotton in general.
b. yarns and synthetic fabrics of nylon and rayon type.
c. yarns and mixed fabrics, made of mixtures of cotton fibres with synthetic fibres
d. yarns and fabrics obtained with other natural polymers different from cotton (i.e. wool, silk, linen, hemp) either alone or mixed with other natural and/or synthetic fibres.
e. "non-woven" according to the definition ISO 9092 : 1988 and CE standard EN 290921) which are used in clothing, home decoration and in the sanitary/health field, for which a precise percentage, as a matter of fact ranging from 1 to 100%, or from 2% to 10% or preferably 3-6% or 5% of the natural of the artificial fibres, in the total formulation are represented by labelled fibres.

The invention will be better detailed in the experimental examples that follow, which do not, however, represent any practical limitation.

### EXPERIMENTAL PART

### Example 1: Coupling carbohydrate tracers to fibres:

The fibre of cotton or nylon is pre-activated and subsequently the previously oxidized carbohydrate tracers were coupled to fibres.
1.1 Four hundred gr of cotton were wetted with water in a reactor under pressure (3 bars). After eliminating excess water, cotton was treated under pressure with 10 Lt of a 0.05 M sodium periodate (NalO4) solution, for 5 hours having the solution continuously circulating through cotton in closed cycle, at a T° of 40 - 45 °C. The thus oxidized cotton was washed thrice with 10 Lt of water.
   After washing, 10 Lt of a 0.5 % solution of lysine, HCl in 0.05 M carbonate/bicarbonate buffer, pH 9.0, were added to oxidized cotton and the reaction was allowed to take place for 2 hours at room T°, having the solution circulating through cotton under pressure. Subsequently, cotton was treated for 30 minutes with 10 Lt of a 0.5 % borohydride sodium solution and it was washed 4 times with 10 Lt of water before being dried in an oven at 110 °C. A cotton- lysine fibre, or, alternatively, a cotton-polylysine fibre was obtained when a 0.01 % polylysine solution was used.

Either maltose or polygalacturonic acid can be coupled. Carbohydrates were previously oxidized.

In the case of maltose, 10 gr were dissolved in 500 mL of NalO4 solution in 0.1 M acetate buffer, pH 5.5 and oxidation was allowed to take place for 1 hour at room T°, after which oxidation was blocked by adding 5 mL of glycerol. In the case of polygalacturonic acid 5 gr of it were used and oxidation was carried out as described in example 1.1.

The reaction was continued by adding the solution of oxidized carbohydrate to the 400 gr of cotton-lysine (or cotton-polylysine) suspended in 10 Lt of 0.05 M carbonate / bicarbonate buffer, pH 9.0, and the reaction was allowed to take place for 2 hours at room temperature. Subsequently, the cotton was treated with 10 Lt of a 0.1 % sodium borohydride (NaBH4) solution, for 30 minutes.

Finally, the cotton was washed 4 times with 10 Lt of water before being dried in a oven at 60 °C.
1.2 Five hundred gr of nylon yarns were pre-activated by treatment with 3.5 N HCl for 16 - 24 hours and washing them subsequently a few times with water.
   Then nylon was resuspended in 10 Lt of 0.05 M carbonate / bicarbonate buffer, pH 9.0, containing DVS (divinilsulfone) at a concentration ranging from 0.01 to 0.5 % with agitation, overnight, at room T°, before being washed three times with 10 Lt water each time. It was then resuspended in 10 Lt of 0.05 M carbonate / bicarbonate buffer, pH 10.0 containing the tracer substance to be coupled, at a concentration ranging from 0.01 to 0.5 % (preferably 1 %) in the case of maltose and at a concentration in the range of 0.1 to 2 % (preferably 0.5 %) in the case of polygalacturonic acid, allowing it to react overnight at room T°. Then, the labelled yarns were washed several times with 10 Lt of water before being dried.

### Example 2: Coupling of aminoacids and polyaminoacids to fibre.

2.1 In the case of gamma-aminobutyric acid or aspartic acid, as in Example 1.1., were added to oxidized cotton 10 Lt of a 0.01 to 0.5 % (as a mean 0.1 %) solution of aminoacid in 0.05 M carbonate / bicarbonate buffer, pH 9.0, and the mixture was allowed to react for one hour at room T°, while having the solution circulating through the fibre. Subsequently, cotton was treated for 30 minutes with 10 Lt of sodium borohydride (NaBH4) solution at a concentration corresponding to that of the tracer substance.
   Successively, cotton was washed four times with 10 Lt of water, before being dried in a oven at 105 °C.
   In the case of polyaminoacid, oxidized cotton, washed as indicated above, was reacted, under pressure and continuous circulation, with 10 Lt of a 0.01 to 0.5 % (as a mean 0.1 %) solution of polylysine (Mr from 1,000 to 10,000) in 0.05 M carbonate / bicarbonate buffer, pH 9.0, for 1 hour at room temperature.
   Subsequently, the cotton was washed four times with 10 Lt of water. The derivatized cotton can be dried or used as such (label = polylysine), or can also be used for coupling another tracer substance either through one of its aminogroups, or through a carboxylic group. For this, derivatized cotton with polylysine was resuspended in 8 Lt of 0.1 M acetate buffer, pH 5.0. To the cotton suspension were added 2 Lt of solution of the tracer substance bearing an amino or a carboxylic group at a concentration between 0.01 and 0.5 % (as an average 1 %) and a quantity in 10 to 40 times in molar excess over the moles of tracer to be coupled of 1-ethyl-3(3-dimethylaminopropyl)carbodiimide (EDAC) in powder. The reaction was allowed to take place for 4 - 6 hours, while agitating, at room T° and the cotton was washed three times with 10 Lt of water, before being dried.
2.2 By contrast, nylon fibre is pre-treated as in example 1.2. and it was resuspended in 10 Lt of 0.05 M acetate buffer, pH 5.0, containing 10 gr of gamma-aminobutyric acid or 10 gr of sodium aspartate.
   Successively, 20 gr or 40 gr respectively, of EDAC in powder were added and the reaction was allowed to take place for 2 hours, under agitation, at room T°, after which fibre is washed four times with 10 Lt of water before being dried at 40 °C.

### Example 3: Coupling carboxylic acid to fibres.

Nylon fibres pre-treated as in example 1.2. and cotton fibres pre-oxidized and coupled to lysine as in example 1.1. were used. Different carboxylic acids can be coupled , such as salicylic acid, m-touic acid, urocanic acid and cholic acid.
3.1 Activated nylon fibre was resuspended in 10 Lt of a 2 : 1 mixture of 0.05 M carbonate / bicarbonate buffer, pH 9.0 and dioxane containing 10 gr of salicylchloride and it was allowed to react under agitation, for 6 hours, at room T°, after which fibre was washed a first time with 10 Lt of the mixture 2 : 1 of carbonate buffer and dioxane and, successively, three times with 10 Lt of water. Drying was carried out in an oven at 40 °C.
   In the case of nicotinic acid, fibre was resuspended in 0.05 M acetate buffer, pH 5.0 containing the tracer to be bound at a concentration ranging from 0.01 to 0.5 % (preferably 0.1 %). Then, an amount of EDAC in powder in an molar excess of 10 to 40 times over the molar concentration of the tracer substance was added.
   Coupling was allowed to take place for 4 - 6 hours at room T° and subsequently fibre was washed 3 times with 10 Lt of water. Eventually, it was dried.
   For m-toluic acid it was operated in the same way using 10 gr of acid and 40 gr of EDAC.
   For coupling of urocanic acid was used 0.05 M carbonate / bicarbonate buffer, pH 9.0 containing DVS (divinilsulfone) at a 0.1 % concentration and the mixture was kept overnight at room T°, after which washing was carried out 3 times with 10 Lt of water. Fibre was then resuspended in 10
   Lt of 0.05 M carbonate / bicarbonate buffer, pH 11.0 containing 10 gr of urocanic acid, allowing it to react overnight at room T°. Labelled yarns were washed three times with 10 Lt of water, before being dried at 40 °C.
   For cholic acid the same reaction schedule was applied, but coupling was performed in 0.05 M carbonate / bicarbonate buffer pH 10.0.

### Example 4: Coupling nicotinamide to fibre.

Fibres of cotton or of nylon were activated as described in examples 1.1. and 1.2.

Cotton was immediately resuspended in 10 Lt of 0.05 M carbonate / bicarbonate buffer, pH 9.0, containing 10 gr of nicotinamide, and reaction was allowed to take place for 2 hours at room T°. Subsequently, it was treated with 10 Lt of a 0.5 % sodium borohydride solution for 30 minutes and it was washed four times with water and dried in an oven at 60 °C.

By contrast, nylon was first resuspended in 10 Lt of 0.05 M carbonate / bicarbonate buffer pH 9.0, containing 0.1 % DVS, kept overnight at room T° and then washed 3 times with water. Subsequently, it was resuspended in 0.05 M carbonate / bicarbonate buffer, pH 9.0, containing 10 gr of nicotinamide, it was washed thrice and dried at 40°C.

### Example 5: Coupling of amino compounds to fibre.

To cotton were coupled 5-aminouracile and 1-naphtylamine, to nylon 6-aminopurine and triaminophenol. Both fibres were pre-treated as in examples 1.1. and 1.2..
5.1 For coupling 5-aminouracile to cotton, the same experimental protocole was used as in the case of nicotinamide (preceding example).
   To couple 1-naphtylamine, cotton-lysine fibre was suspended in 10 Lt of 0.05 M carbonate / bicarbonate buffer, pH 9.0, to which were added 40 mL of a 25 % glutaraldehyde solution. It was allowed to react at room T° for 2 hours and four washes were carried out with 10 Lt of water.
   Subsequently, it was resuspended in 10 Lt of 0.05 M carbonate / bicarbonate buffer, pH 9.0, and 10 gr of naphtylamine dissolved in 500 mL of water were added. It was allowed to react at room T°, for 2 hours, and four washes were carried out with 10 Lt of water and the material is dried at 105 °C.
5.2 To couple 6-aminopurine or 5-amino-uracile, nylon was rapidly treated with 0.5 N NaOH and the excess NaOH is eliminated by filtration.
   Fibre is resuspended in 5 Lt of a solution of cyanuric chloride in acetone at a concentration from 0.01 to 0.5 % and 10 Lt of water were added.
   After contact for 15 seconds acetic acid is added at 20 % final concentration, and, then, fibre was washed with a 1 : 3 mixture of acetone : water. Successively, it was suspended in 5 Lt 0.1 M phosphate buffer, pH 7.0, containing the amino tracer substance at a concentration ranging from 0.01 to 0.5 % (preferably 0.1%). After 15 minutes of reaction, 1 M NH40H / NH4Cl buffer, pH 8.6, was added in a quantity equivalent to that of cyanuric chloride and fibre was dried.
   Coupling of triaminophenol was performed in the same way as for urocanic acid (example 3.2.), with the difference that carbonate buffer exhibits a pH of 10.0.
   If 1-naphtylamine had to be coupled to fibre, this latter was suspended in 10 Lt of a 0.02 M Na2HP04 solution, pH 8.2 and 10 gr of glutaraldehyde (40 mL of the 25% solution) were added allowing contact to occur for 2 hours at room T°. Then, activated fibre was washed 3 times with 10 Lt of water, after which it was resuspended in 10 Lt of 0.05 M carbonate buffer, pH 9.0. Ten gr. of 1-naphtylamine were then added and allowed to react for 2 hours at room T°.
   Labelled fibre was washed thrice with 10 Lt of water and it was dried in an oven at 40°C.

### Example 6: Coupling of anthraldehyde to fibre.

Cotton and nylon pre-treated as described in the preceding examples were used. Fibres were resuspended, respectively, in 10 Lt of a 2 : 1 mixture of 0.05 M carbonate buffer, pH 9.0 and DMSO (dimethylsulfoxide) for cotton and of only buffer for nylon; then, 10 gr of anthraldehyde dissolved in 500 mL of DMSO were added and the reaction was allowed to take place, respectively, for 5 or 4 hours at room T°.

Cotton was subsequently washed with 10 Lt of a 2 : 1 mixture of water : DMSO and, subsequently, 3 times with 10 Lt of water before being dried in an oven at 60 °C; nylon, in contrast, was directly washed four times and dried at 40 °C.

### Example 7: Coupling of sulfhydryl compounds (sodium 2-mercapto-5-benzimidazole-sulfonate) to fibre.

Pre-treated cotton and nylon fibres were resuspended in 10 Lt of 0.05 M carbonate / bicarbonate buffer, pH 11.0 (for cotton) and pH 9.0 (for nylon) containing DVS (divinylsulfone) at 0.1 %.
Contact was allowed to occur overnight, under agitation, at room T°, after which, in the case of nylon three washings with 10 Lt of water, each time, were carried out. Subsequently, fibres were resuspended in 10 Lt of 0.05 M carbonate / bicarbonate buffer, pH 8.0, containing 10 gr of mercapto-derivative allowing it to react overnight at room T°.
Finally, the labelled fibre was washed several times with 10 Lt of water and was successively dried in a oven, respectively, at 60 °C and at 40 °C.

### Example 8: Coupling of purine bases (6-methyl-purine) to fibre.

Was used only cotton fibre pre-treated as described in example 1.1. and derivatized with lysine or polylysine. To cotton-lysine or polylysine suspended in 10 Lt of a 9 : 1 mixture of tetrahydrofurane : water, at pH 10.0 were added 10 gr of trichloro-methylpurine in 1 Lt of tetrahydrofurane and the mixture was kept in contact, under pressure, for 2 hours, at room T°. Subsequently, cotton was washed with 10 Lt of a 9 : 1 mixture of tetrahydrofurane : water and successively thrice with 10 Lt of water each time, before being dried in an oven at 60 °C.

### Example 9: Coupling of polyamine to fibre.

400 gr cotton were wetted with water in a reactor, under pressure (3 bars). Successively, after removing excess water, cotton was treated, under pressure with 10 Lt of a DVS (divinylsulfone) solution at a concentration ranging from 0.01 to 0.5 % in 0.05 M carbonate / bicarbonate buffer, pH 11.0, and cotton activation was allowed to occur overnight, at room T°. Then, cotton was washed 3 times with 10 Lt of water. The thus activated cotton was treated with 10 Lt of a solution of triaminophenol in 0.05 M carbonate / bicarbonate buffer, pH 10.0.

After reaction for six hours at room T°, cotton was washed 3 times with 10 Lt of water and then dried. The obtained derivative can be, on turn, used as an intermediate for coupling other typologies of tracers.

Tracer substances can be coupled to this intermediate utilizing: the reaction with carbodiimide (EDAC) when -COOH groups are present; the reaction with aldehyde groups (if the tracer substance can be oxidized); the presence of halogen radicals (Cl, Br, F).

### Example 10: Synthesis of maltose immunogen

Thirty-five mg of BSA (bovine albumin - Sigma A 4503) are dissolved in 4 mL of 0.1 M carbonate buffer pH 10.0 and 1.4 mL of DVS are added; the mixture was agitated overnight at room T°. The mixture was subsequently filtered through a Sephadex G 100 column using 10 mM phosphate buffer pH 7.2 as eluent. Fractions containing DVS-BSA were collected with a yield around 17 mg and were then pooled and dialyzed against 0.1 M carbonate buffer pH 10.0. To the dialyzed mixture were then added 2.5 mg of maltose in powder and coupling was allowed to take place, under agitation, overnight at room T°, after which the reaction product was dialyzed in a dialysis tubing with 10kD breakthrough point against PBS (phosphate buffer saline) at 4 °C.

BSA-maltose representing the immunizing immunogen was obtained (which was administered to animals to induce the formation of antibodies).

Similarly, but using OVA (ovalbumin) was obtained the control immunogen (used during the course of experiments to check the antibody response).

### Example 11: Synthesis of polygalacturonic acid immunogen

First, tracer substance was oxidized. Fifty mg of polygalacturonic acid were dissolved in 50 mL of NaIO4 solution (sodium periodate) in 0.1 M acetate buffer pH 5.5, and oxidation was allowed to occur, under agitation, in the dark, for 1 hour at room T° and the reaction mixture was dialyzed in a dialysis tubing with 10 kD cut-off, for 5 hours against distilled water and overnight against 0.1 M carbonate / bicarbonate buffer, pH 9.0. Oxidized polygalacturonic acid was then added to a solution containing 70 mg of BSA in 1 mL of saline and the mixture was kept overnight at 4 °C, after which it was dialyzed against PEG 20000 solution until its volume is concentrated down to 10 mL and, successively, dialyzed against PBS (phosphate buffer saline). Was obtained: BSA-polygalacturonic acid = immunizing immunogen. Similarly, with OVA was obtained the control immunogen.

### Example 12: Synthesis of 1-naphtylamine immunogen

Fifty mg of KLH (Keyhole Limpet Hemocyanin) dissolved in 5mL of 0.01 M PBS, pH 7.2, were dialyzed against 200 mL of 0.2 % glutaraldehyde solution in the same buffer, overnight at 4 °C. The thus activated KLH was further dialyzed against 500 mL of PBS - 3 treatments each of 3 hours. After dialysis, to activated KLH were added 5 mg of 1-naphtylamine, HCl dissolved in 1 mL of water. pH of the solution was adjusted to 7.2 and coupling was allowed to take place overnight at 4 °C, after which reactive groups still free were blocked by adding 0.1 mL of a 0.2 M lysine, HCl solution. After reaction for 1 hour, the mixture was dialyzed against PBS. Is obtained KLH-naphtylamine-immunizing immunogen. Similarly, using a second protein (i.e. BSA) in the same molar ratio was obtained the control immunogen.

### Example 13: Synthesis of aspartic acid immunogen

To 100 mg of KLH dissolved in 5 mL of 0.1 M acetate buffer, pH 5.5, were added 10 mg of sodium aspartate in powder (aspartic acid : KLH ratio = 25 : 1). When aspartate was dissolved, 25 mg of EDAC were added and the reaction was allowed to take place, under agitation, for 4 hours at room T°, while readjusting the pH to 5.5 during the first 30 minutes of reaction. After completion of the reaction, the mixture was dialyzed against PBS. Was obtained: KLH-aspartate-immunizing immunogen. Similarly, using a second protein (for instance, BSA) was obtained the control immunogen.

### Example 14: Synthesis of salicylic acid immunogen

A mixture was prepared by dissolving 14 gr of salicylic acid in 11 gr of thionylchloride and 0.14 gr of anhydrous pyridine. Successively, the mixture was concentrated and the concentrated salicylchloride residue was recristallized from ethanol. To 1 gr of BSA dissolved in 10 mL of a 3 : 1 mixture of 0.1 M carbonate buffer, pH 9.0, and dioxane, 100 mg of salicylchloride dissolved in 2 mL of dioxane were added and the reaction was allowed to take place overnight, at room T°, in the dark, after which the mixture was dialyzed against PBS. The control immunogen was prepared as described in the previous Example.

### Example 15: Synthesis of triaminophenol immunogen

Hundred mg of KLH were dissolved in 5 mL of 0.1 M carbonate buffer, pH 10.0, and 0.7mL of DVS were added; reaction was allowed to occur overnight, at room T°. Then the mixture was dialyzed against few changes of 0.1 M carbonate buffer, pH 10.0. To the dialyzed DVS-KLH mixture was added 10 mg of triaminophenol dissolved in 1 mL of dioxane and reaction was allowed to take place overnight, at room T°, after which the mixture was dialyzed against PBS (phosphate buffer saline).

### Example 16: Synthesis of nicotinic acid immunogen

Hundred mg of KLH were dissolved in 5 mL of 0.1 M acetate buffer, pH 5.5. To this solution 10 mg of acid was added and immediately afterwards 50 mg of EDAC in powder. The mixture was agitated for 4 hours, at room T°, while readjusting the pH to 5.5 during the first 30 minutes. After completion of the reaction, the mixture was dialyzed against PBS.

### Example 17: Synthesis of nicotinamide immunogen

Hundred mg of KLH were dissolved in 5 mL of 0.05 M carbonate buffer, pH 9.0, and 10 mg of nicotinamide dissolved in 1 mL of water and 20 mg of glutaraldehyde were successively added. Reaction was allowed to occur for 2 hours, at room T° and subsequently, overnight at 4 °C. Then, the reaction was blocked by adding 0.1 mL of 2 M glycine solution and the mixture was dialyzed against PBS.

### Example 18: Synthesis of 6-methylpurine immunogen

Fifty mg of 6-methylpurine dissolved in 10 mL of pyridine were treated with 100 mg of SO2Cl2 in an ice-bath. After adding sulfonylchloride, the mixture was heated on reflux for 30 minutes before being brought to dry. The thus formed trichloromethylpurine was redissolved in 5 mL of a 9 : 1 mixture of tetrahydrofurane : water, at pH 10.0, and 100 mg of BSA dissolved in 5 mL of the same mixture were added. After agitating the reaction mixture for 1 hour, at room T°, it was dialyzed against PBS.

### Example 19: Synthesis of 9-anthraldehyde immunogen

About 100 mg of KLH were dissolved in a 2 : 1 mixture of 0.05 M carbonate buffer, pH 9.0, and dioxane. Ten mg of anthaldehyde dissolved in 0.5 mL of dioxane were added and reaction was allowed to take place for 2 hours at room T° and, successively, overnight at 4 °C. The reaction product was then dialyzed for 4 hours against a 2 : 1 mixture of PBS : dioxane and overnight against PBS.

### Example 20: Synthesis of toluic acid immunogen

Hundred mg of KLH are dissolved in 5 mL of 0.05 M acetate buffer, pH 5.0, and 14 mg of sodium toluate dissolved in 1 mL of acetate buffer were added to KLH solution and immediately afterwards 50 mg of EDAC in powder. The mixture was agitated for 4 hours, at room T°, while readjusting the pH to 5.0 during the first 30 minutes. After reaction, the mixture was dialyzed against PBS.

### Example 21: Synthesis of urocanic acid immunogen

To 100 mg of KLH dissolved in 5 mL of water 2.5 mL of a 3 M sodium acetate solution and 5 mL of a 8 % formaldehyde solution were added. To this mixture 10 mg of urocanic acid dissolved in 1 mL DMSO (dimethylsulfoxide) were added and agitated continuously overnight at room T°. Then, the reaction mixture was dialyzed against a 3 : 1 mixture of PBS : DMSO and finally against PBS.

### Example 22: Synthesis of cholic acid immunogen

To 100 mg of KLH dissolved in 5 mL of 0.05 M acetate buffer, pH 5.0, were added 7.6 mg of sodium cholate dissolved in 1 mL of the same buffer and 50 mg of EDAC in powder. The mixture was allowed to react for 4 hours at room T°, while readjusting the pH to 5.0 during the first 30 minutes after which was dialyzed against PBS.

Control immunogen was prepared differently: 100 mg of BSA are dissolved in 10 mL of 0.1 M carbonate buffer, pH 10.0, and 1 mL of DVS is added. The mixture was allowed to react, overnight, at room T°, and it was subsequently dialyzed for 4 hours against 500 mL of the same buffer solution. To the dialyzed solution of BSA, 10 mg of sodium cholate were added and reaction was allowed to take place for 3 hours at room T° or also overnight at 4 °C. The mixture was eventually dialyzed against PBS.

### Example 23: Synthesis of 6-aminopurine and of 5-aminouracile immunogens

Hundred mg of KLH dissolved in 10 mL of PBS were dialyzed against 200 mL of a 0.2 % glutaraldehyde solution in 0.01 M PBS, pH 7.2, for 16 hours, at 4 °C, after which the solution was dialyzed 3 times for 3 hours against PBS. To the activated KLH solution, 9 mg of 6-aminopurine dissolved in 9 mL of water were added and the mixture was agitated for 16 hours at 4°C, after which the reaction was blocked by the addition of 0.1 mL of 2 M glycine and the mixture dialyzed against PBS.

### Example 24: Synthesis of 2-mercapto-5-benzimidazole sulfonate immunogen

To a mixture of 100 mg KLH and 9.9 mg of the sulfonate compound in 10 mL of 0.02 M disodium phosphate solution, pH 8.2, 20 mg of HCHO were added under the form of 278 uL of a dilution of the 36 % solution. The mixture was incubated for 24 hours in an oven at 37 °C, before being dialyzed against PBS.

After preparing the immunogens according to examples 10 - 24, the antibodies were produced using known procedures for immunizing animals. The antibodies were purified using methodologies described, for instance, in: "Antibodies: A Laboratory Manual", 1988, CSH ed., or also in: R. Scopes, "Protein Purification, Principles and Practice", 1982, Springer Verlag Publ., NY., and they were labelled with a revealing enzyme in the case a direct ELISA system was used.

### Example 25: Label identification on fibres, yarns, fabrics, non-wovens.

It was performed on samples of fabrics or of non-wovens in which the labelled fibres were mixed to the non-labelled ones in a 0.3 : 100 and 1 : 100 ratio (for fabrics)and 5 :100 ratio (for non-wovens).

The yarns and fabrics were subjected to normal processing treatments. The non-wovens for sanitary use were prepared employing the "wetlaid" process technique, whereas for clothing the "drylaid" technique was applied. Revelation of the label was performed by the indirect ELISA method in which the enzyme peroxidase (HRP) was bound to the second antibody, while the enzyme substrate was tetramethylbenzidine (TMB). The quantitative determination was carried out by measuring spectrophotometrically (at 370 nm) the intensity of the coloration generated in the liquid by fragments of fabrics / non-wovens immersed into it. For qualitative determination as performed visually, has been used a subjective scale: (-) = no coloration; (+) = slightly coloured; (++) = fairly coloured; (+++) = strongly coloured, with reference to standard solutions of increasing colour. For checking the specificity of the method have been used as controls fragments of fabrics / non-wovens not treated. Results are reported in the following tables.

| **25.1.** Identification of label on cotton fibres /yarns / fabrics | | | | |
|---|---|---|---|---|
| Tracer substance | Revelation | | | |
| | Qualitative Label percentage | | Quantitative (370 nm) | |
| | 0,3% | 1% | 0,3% | 1% |
| Not treated 0.025 | ( - ) | ( - ) | | 0.045 |
| Maltose | ( ++ ) | ( +++ ) | 0.420 | 0.790 |
| Polygalacturonic acid | ( +++ ) | ( +++ ) | 0.848 | 0.980 |
| Gamma-aminobutyric acid | ( +++ ) | ( +++ ) | 0.650 | 0.840 |
| Aspartic acid | (++ ) | ( +++) | 0.350 | 0.770 |
| m-toluic acid | ( ++ ) | ( +++ ) | 0.280 | 0.680 |
| Urocanic acid | ( ++ ) | ( +++ ) | 0.400 | 0.860 |
| Cholic acid | ( ++ ) | ( ++ ) | 0.240 | 0.410 |
| Salicylic acid | ( ++ ) | ( +++ ) | 0.350 | 0.820 |
| Nicotinamide | ( ++ ) | ( ++ ) | 0.240 | 0.380 |
| 6-methylpurine | ( ++ ) | ( ++ ) | 0.360 | 0.740 |
| 5-aminouracile | ( ++ ) | ( +++ ) | 0.220 | 0.680 |
| 1-naphtylamine | ( +++ ) | ( +++ ) | 0.690 | 0.920 |
| Triaminophenol | ( +++ ) | ( +++ ) | 0.840 | 1.050 |
| 9-anthraldehyde | ( ++ ) | ( +++ ) | 0.300 | 0.580 |
| 2-mercaptobenzimidazole | ( ++ ) | ( +++ ) | 0.320 | 0.720 |

| **25.2** Label identification on nylon fibres / yarns/ fabrics | | | | |
|---|---|---|---|---|
| Tracer substance | Revelation | | | |
| | Qualitative Label percentage | | Quantitative (370 nm) | |
| | 0,3% | 1% | 0,3% | 1% |
| Not treated | ( - ) | ( - ) | 0.030 | 0.032 |
| Maltose | ( +++ ) | ( +++ ) | 0.750 | 0.900 |
| Polygalacturonic acid | ( +++ ) | ( +++ ) | 0.720 | 0.940 |
| Gamma-aminobutyric acid | ( +++ ) | (+++ ) | 0.670 | 0.890 |
| Aspartic acid | ( +++ ) | ( +++ ) | 0.630 | 0.860 |
| Nicotinic acid | ( +++ ) | ( +++ ) | 0.740 | 0.990 |
| m-toluic acid | ( +++ ) | ( +++ ) | 0.610 | 0.780 |
| Urocanic acid | ( +++ ) | ( +++ ) | 0.560 | 0.830 |
| Cholic acid | ( +++ ) | ( +++ ) | 0.600 | 0.930 |
| Salicylic acid | ( +++ ) | ( +++ ) | 0.650 | 0.990 |
| Nicotinamide | ( ++ ) | ( +++ ) | 0.280 | 0.620 |
| 6-aminopurine | ( +++ ) | ( +++ ) | 0.500 | 0.870 |
| 5-aminouracile | ( +++) | ( +++ ) | 0.470 | 0.800 |
| 1-naphtylamine | ( +++ ) | ( +++ ) | 0.740 | 1.030 |
| Triaminophenol | ( +++ ) | ( +++ ) | 0.880 | 1.230 |
| 6-methylpurine | ( +++ ) | ( +++ ) | 0.590 | 0.900 |
| 9-anthraldehyde | ( ++ ) | ( +++ ) | 0.310 | 0.650 |
| 2-mercaptobenzimidazole | ( +++ ) | ( +++ ) | 0.490 | 1.020 |

| **25.3.** Label identification on various kinds of non-wovens in which the percentage of labelled fibre is 5 %. The labelled fibre is that of cotton. | | | |
|---|---|---|---|
| Composition | Tracer substance | Revelation | |
| | | Qualitative | Quantitative (370 nm) |
| a) Chitin/polyester 50 % Cotton 50 % (for sanitary use) | 1-naphtylamine | ( +++ ) | 0.900 |
| b) Polyester / Cotton 50 % 50 % (for underwear) | triaminophenol | ( +++ ) | 0.850 |
| | m-toluic acid | (+++ ) | 0.950 |
| c) Polyester / Cotton 50 % 50 % (clothing articles) | triaminophenol | ( +++ ) | 0.940 |
| | m-toluic acid | ( +++) | 0.900 |
| d) Polyester / Cotton 50 % 50 % | triaminophenol | (+++) | 1.020 |
| | m-toluic acid | (+++) | 0.980 |
| (interiors) | | | |

| **25.4**. Identification of a double label on cotton fibres/ yarns/fabrics The method, the enzyme and the substrate are the same as those of the former examples: Were used fibres/yarns/fabrics containing two different labels. The revelation was carried out in two successive steps (1st and 2nd) using each time specific antibodies for the different tracer substances. As an example has been described the double labelling but it was also possible to use a larger number of tracer substances. The use of such a technique was particularly useful in the case of fibres and mixed yarns and it offers a major guarantee against the possible risk of sophistication. | | | | |
|---|---|---|---|---|
| Sample | % of tracer substance | Response to specific Ab Ab-anti 1 st tracer / Ab anti 2nd tracer | | |
| 1 ° maltose 0.3% aspartate 0.3% | | 1 s^{t} | +++ | --- |
| | | 2^{nd} | --- | +++ |
| 2° salicylic 0.3% / 1 -naphtylamine 0.3% | | 1^{st} | +++ | --- |
| | | 2nd | --- | +++ |
| 3° maltose 0.3% / triaminophenol 0.3% | | 1 st | +++ | --- |
| | | 2nd | --- | +++ |
| 4° nicotinamide 0.3% / aspartic 0.3% | | 1 st | +++ | --- |
| | | 2^{nd} | --- | +++ |
| 5° cholic acid 0.3% / 6-methylpurine 0.3% | | 1^{st} | +++ | --- |
| | | 2nd | --- | +++ |

## Claims

1. Fibres labelled with identification tracers **characterized by** the fact that said tracer substances are selected from the group consisting of: aminoacids, carbohydrates, amines and polyamines, amides, aldehydes, organic acids, purine bases, sulfhydryl derivatives.

2. Fibres according to claim 1) wherein the carbohydrates are maltose and polygalacturonic acid; the aminoacids are GABA (gamma-aminobutyric acid) and DL-aspartic acid; the carboxylic acids are nicotinic acid, m-toluic acid, urocanic acid, salicylic acid, cholic acid; the amino compounds are 1-naphtylamine, 5-aminouracile, 6-aminopurine, triaminophenol; the purine bases are 6-methylpurine; the thiol derivatives are 2-mercapto-benzimidazole; the aldehydes is 9-anthraldehide; the amides, nicotinamide.

3. Fibres according to claim 1) wherein the tracer substances are covalently bound to the fibre.

4. Fibres according to claim 3) wherein the coupling of a tracer substance to a fibre is either direct or it takes place through a chemical bridge.

5. Fibres according to claim 4) wherein the bridge is a polyaminoacid, a polyamine, an aminopolycarboxylic or polyacid structure.

6. Fibres according to claim 5) wherein said polyaminoacid bridge is selected from: polylysine, polyglutamic or polyaspartic acids.

7. Fibres according to claim 5) in wherein the polyamine bridge is triaminophenol.

8. Fibres according to claims 1 - 7) wherein the fibres are natural or synthetic.

9. Fibres according to claim 8) wherein natural fibres are chosen in the group consisting of: cotton, linen, hemp, wool and silk and synthetic fibres are chosen in the group costituted by: naylon, rayon.

10. Yarns, fabric, non-woven comprising the labelled fibres according to claim 8).

11. Yarns, fabrics and non-woven according to claim 9) wherein labelled fibres are mixed or homogenized with non-labelled fibres.

12. Yarns and fabrics according to claim 11) wherein the labelled fibres represent from 0.01 to 10% of the total fibres.

13. Yarns and fabrics according to claim 12) wherein the labelled fibres represent from 0.1 to 5 % of the total fibres.

14. Yarns and fabrics according to claim 13) wherein the labelled fibres represent from 1 to 3 % of the total fibres.

15. Yarns and fabrics according to claim 14) wherein the labelled fibres represent at least 1 % of the total fibres.

16. Non-woven according to claim 10) wherein the labelled fibres represent from 1 to 10 % of total fibres.

17. Non-woven according to claim 16) wherein the labelled fibres represent from 2 to 7 % of total fibres.

18. Non-woven according to claim 17) wherein the labelled fibres of non-woven represent 2.5 to 6 % of total fibres.

19. Non-woven according to claim 18) wherein the labelled fibres represent at least 3 % of total fibres.

20. Method for labelling fibres with GNO identification tracer comprising an activation of the fibres, followed by coupling of tracer substances to the activated fibres, **characterized in that** such tracers are selected from the group consisting of: aminoacids, carbohydrates, amines and polyamines, amides, aldehydes, organic acids, purine bases, thiols.

21. Method according to claim 20) wherein carbohydrates are: maltose and polygalacturonic acid; aminoacids are GABA (gamma-aminobutyric acid) and DL-aspartic acid; the carboxylic acids are nicotinic acid, m-toluic acid, urocanic acid, salicylic acid, cholic acid; the amino compounds are 1-naphtylamine, 5-aminouracile, 6-aminopurine, triaminophenol; the purine bases is 6-methylpurine; the thiol compounds are 2-mercaptobenzimidazole; the aldehydes is 9-anthraldehyde; the amides, nicotinamide.

22. Method according to claim 21) wherein the fibres are natural or synthetic.

23. Method according to claim 22) wherein natural fibres are selected from the group consisting of: cotton, linen, hemp, wool and silk synthetic fibres are selected from the group consisting of: nylon, rayon.

24. Method according to claim 22) wherein the activation of natural fibres is performed by a periodic oxidation.

25. Method according to claim 22) wherein the activation of synthetic fibres is performed by an acid treatment.

26. Method according to claim 20 - 25) wherein coupling of the tracer substance to the fibre is direct or also takes place through a linking bridge.

27. Method according to claim 26) wherein the linking bridge is a polyaminoacid, polyamine, aminopolyacid or a polyacid.

28. Method according to claim 27) wherein the polyaminoacid linking bridge is selected from polylysine, polyglutamic or polyaspartic acid.

29. Method according to claim 27) wherein the polyamine linking bridge is triaminophenol.

30. Method for detection of fibres labelled with identification tracers, yarns, fabrics, non-wovens according to claim 1 - 19) **characterized in that** the detection is performed by an immunochemical reaction between the identification tracers coupled to fibres and specific antibodies.

31. Method according to claim 30) wherein said immunochemical reaction is an immunoenzymatic reaction.

32. Method according to claims 30) and 31) wherein tracer substances are selected from the group consisting of aminoacids, carbohydrates, amines and polyamines, amides, aldehydes, organic acids, purine bases and thiol derivatives.

33. Method according to claim 32) wherein carbohydrates are maltose, polygalacturonic acid; the aminoacids are GABA (gamma-aminobutyric acid) and DL-aspartic acid; the carboxylic acids are nicotinic acid, m-toluic acid, urocanic acid, salicylic acid, cholic acid; the amino compounds are 1-naphtylamine, 5-aminouracile, 6-aminopurine, triaminophenol; the purine bases is 6-methylpurine; the sulfhydryl derivatives is 2-mercaptobenzimidazole; the aldehyde is 9-anthraldehyde; the amide nicotinamide.

34. Method according to claim 33) wherein the detection takes place by immersion of fibres, yarns, fabrics, non-woven into a solution.

35. Method according to claim 34) wherein the solution contains a substrate for an enzyme.

36. Method according to claim 35) wherein the enzyme is selected from the group consisting of: peroxidase, alkaline phosphatase and beta-galactosidase.

37. Method according to claim 36) wherein the enzyme reacts with a chromogenic substrate.

38. Method according to claim 37) wherein the enzyme is linked to an antibody.

39. Method according to claims 37 - 38) for a qualitative and/or a quantitative detection of the label.

40. Method according to claims 30 - 39) wherein the detection is colorimetric.

41. Method according to claims 37 - 40) wherein said qualitative or quantitative detection is carried out by a spectrophotometric reading of the solution.

42. Kit for the detection of labelled fibres, yarns fabrics and non-woven according to the method of claims 30 - 41) comprising a test tube with a solution containing an antibody specific for comprising an identification tracers and a test tube containing an antibody coupled with an enzyme and directed to the first antibody with an additional test tube comprising of a solution containing the substrate for the enzyme, and optionally, buffers, detergents and reference standard solutions.
